# EUROPEAN PATENT APPLICATION

(11) **EP 3 435 381 A1**
(43) Date of publication of application: **30.01.2019**
(21) Application number: 18185622.0
(22) Date of filing: 25.07.2018
(51) Int. Cl.: G16H 20/30, G16H 40/63, G16H 40/67, A61H 7/00

(54) **MASSAGE MACHINE SYSTEM, MASSAGE MACHINE USED THEREIN, AND WEARABLE MEASRUREMENT INSTRUMENT USED THEREIN**

(30) Priority: 26.07.2017 JP 2017144968
(71) Applicant: Family Inada Co., Ltd., Osaka 532-0004 (JP)
(72) Inventor: INADA, Nichimu, Osaka-shi, Osaka 532-0004 (JP); ISHIDOU, Yuta, Saihaku-gun Tottori, Tottori 689-3224 (JP)
(74) Representative: Horn Kleimann Waitzhofer Patentanwälte PartG mbB

(57) **Abstract**

There is provided a massage machine system capable of performing proper massage by using physical information such as a pulse rate acquired by a wearable measurement instrument. The massage machine system includes a massage machine that includes a massage unit for massaging a body of a user and a control unit for controlling an operation of the massage unit, a wearable measurement instrument that has a detection unit for detecting physical information of the user and a transmission unit capable of transmitting the physical information detected by the detection unit, a server for storing the physical information detected by the wearable measurement instrument, and a portable electronic information communication terminal. The server is communicably connected to the massage machine and the electronic information communication terminal, respectively via a public network. The electronic information communication terminal includes a receiving unit capable of receiving the physical information transmitted from the transmission unit.

## Description

### Technical Field

The present invention relates to a massage machine system including a massage machine for massaging a user, and a massage machine and a wearable measurement instrument which are used therein, and particularly relates to a massage machine which can perform proper massage by using physical information such as a pulse rate or a step count acquired by a wearable measurement instrument.

### Background Art

A chair type or mattress type massage machine is widely known. For example, the chair type massage machine has a seat unit and a backrest unit and employs a structure in which a treatment unit for performing massage is disposed in the backrest unit. Then, a plurality of built-in motors are driven in combination with each other. In this manner, the treatment unit can selectively perform a plurality of operations (for example, a kneading operation, a patting operation, and changing a treatment target site). The massage machine which can perform the plurality of operations in this way has a controller and is operated by a user using the controller. In this manner, the user selects a massage course (a relaxation course, a stress relief course, or a professional course) corresponding to a predetermined combination of movements of the treatment unit and pressure applying operations.

Incidentally, a type of the massage course suitable for the user varies depending on a health condition of the user. Therefore, a massage machine has been proposed which includes a detection device for measuring biological information such as a heart rate, a blood pressure value, a body temperature, and a galvanic skin reflex of the user so as to select the massage course, based on the measured biological information (for example, refer to Patent Documents 1 and 2).

### Related art Document

### Patent Document

Patent Document 1: JP-A-9-192186
Patent Document 2: JP-A-2000-070373

### Summary of the Invention

### Problem that the Invention is to Solve

However, the biological information measured in the massage machine as described above is obtained by a detection device included in the massage machine. Accordingly, the biological information is obtained through only measurement values when the massage machine is used. The measurement values are not perceived when the massage machine is not in use, such as during the user's exercise or sleep.

Therefore, it is not possible to recognize a real health condition of the user, which is perceived using the measurement values when the massage machine is not in use, such as during the user's exercise or sleep, thereby causing a problem in that a proper massage course cannot be selected.

On the other hand, various wearable measurement instruments are on sale. This wearable measurement instrument (for example, a wearable watch equipped with a measurement instrument) is used so that the user acquires a pulse rate during the user's exercise or sleep.

However, even though this pulse rate acquired during the user's exercise or sleep is important information in order to recognize the real health condition of the user, the information has not been used at all for massage (particularly in selecting the massage course).

Therefore, the present invention is made in view of the above-described problem, and an object thereof is to provide a massage machine system which can perform proper massage by using physical information such as a pulse rate acquired from a wearable measurement instrument, and a massage machine and a wearable measurement instrument which are used for the massage machine system.

### Means for solving the problem

In order to achieve the above-described object, a massage machine system according to the present invention includes a massage machine that includes a massage unit for massaging a body of a user and a control unit for controlling an operation of the massage unit, a wearable measurement instrument that has a detection unit for detecting physical information of the user and a transmission unit capable of transmitting the physical information detected by the detection unit, a server for storing the physical information detected by the wearable measurement instrument, and a portable electronic information communication terminal. The server is communicably connected to the massage machine and the electronic information communication terminal, respectively via a public network. The electronic information communication terminal includes a receiving unit capable of receiving the physical information transmitted from the transmission unit.

According to this configuration, for example, the user can acquire the physical information during the user's exercise or sleep for 24 hours, merely by wearing the wearable measurement instrument on the user's body. Based on the acquired physical information, the user can select a proper massage course (such as a stress relief treatment, a fatigue recovery treatment, a stiff shoulder treatment, an insomnia treatment, a blood pressure / edema treatment, or a slimming treatment) when the user receives the massage. For example, based on the acquired physical information, the massage machine may be able to select or recommend the proper massage course. Alternatively, based on the acquired physical information, the user himself or herself may be able to select the proper massage course (for example, the user himself or herself selects the stress relief treatment in a case where the user determines that a stress value is high based on the acquired physical information).

In addition, in the massage machine system according to the present invention, it is preferable that the massage machine includes a receiving unit capable of receiving the physical information transmitted from the transmission unit, and when the transmission unit of the wearable measurement instrument is located within a communicable distance with the receiving unit of the massage machine, the physical information is output to the receiving unit of the massage machine. It is preferable that when the transmission unit of the wearable measurement instrument is located outside a communicable distance with the receiving unit of the massage machine, the physical information is output to the receiving unit of the electronic information communication terminal.

According to this configuration, an output destination of the physical information acquired by the wearable measurement instrument can be changed in accordance with the communicable distance. Specifically, when the wearable measurement instrument and the massage machine are located within the communicable distance, the physical information is output to the receiving unit of the massage machine. When the wearable measurement instrument and the massage machine are located outside the communicable distance, the physical information is output to the receiving unit of the electronic information communication terminal. In addition, for example, the user can acquire the physical information during the user's exercise or sleep for 24 hours while the user wears the wearable measurement instrument on the user's body. Based on the acquired physical information, the user can select the proper massage course when the user receives the massage.

In the above-described invention, it is preferable that the communicable distance is 10 meters.

According to this configuration, an output destination (the massage machine or the electronic information communication terminal) of the physical information can be properly determined.

In addition, a massage machine system according to the present invention includes a massage machine that includes a massage unit for massaging a body of a user and a control unit for controlling an operation of the massage unit, a wearable measurement instrument that has a detection unit for detecting physical information of the user and a transmission unit capable of transmitting the physical information detected by the detection unit, and a server for storing the physical information detected by the wearable measurement instrument. The server is communicably connected to the massage machine via a public network. The massage machine includes a receiving unit capable of receiving the physical information transmitted from the transmission unit.

According to this configuration, for example, the user can acquire the physical information during the user's exercise or sleep for 24 hours, merely by wearing the wearable measurement instrument on the user's body, and can cause the wearable measurement instrument to store the physical information. The stored physical information is output to the massage machine. In this manner, the user can select the proper massage course (such as a stress relief treatment, a fatigue recovery treatment, a stiff shoulder treatment, an insomnia treatment, a blood pressure / edema treatment, or a slimming treatment) when the user receives the massage.

In the above-described invention, it is preferable that the massage machine and/or the electronic information communication terminal transmit the received physical information to the server. It is preferable that the server prepares health information, based on the physical information, and transmits the health information to the massage machine.

According to this configuration, for example, in a case where the server is a computer installed in a selling agency of the massage machine, the physical information received by the massage machine and/or the electronic information communication terminal is transmitted to the server. In this manner, the user can acquire the health information based on the physical information prepared by the server. As a result, the user can more properly select the massage course when the user receives the massage.

In the above-described invention, it is preferable that the server prepares the health information of the user, based on the stored physical information.

According to this configuration, the server can prepare the proper health information.

In the above-described invention, it is preferable that the health information includes an advice for diseases and/or lifestyle habits.

According to this configuration, when the user receives the massage, the user can select the proper massage course including the advice for the diseases and the lifestyle habits (meals and exercise).

In the above-described invention, it is preferable that the physical information represents at least one type of information selected from the group consisting of a pulse rate, a step count, a walking distance, a blood pressure, a body temperature, a sweat rate, a blood glucose level, calorie consumption, a sleep state, a body weight, a body fat percentage, moisture content, muscle mass, bone mass, a basal metabolic rate, BMI, and an obesity index.

According to this configuration, when the user receives the massage, the user can select the proper massage course, based on the pulse rate, the step count, the walking distance, the blood pressure, the body temperature, the sweat rate, the blood glucose level, the calorie consumption, the sleep state, the body weight, the body fat percentage, the moisture content, the muscle mass, the bone mass, the basal metabolic rate, the BMI, and the obesity index.

In the above-described invention, it is preferable that the massage machine system further includes a portable second electronic information communication terminal that is provided for a third party. It is preferable that the second electronic information communication terminal and the server are communicably connected to each other via the public network. It is preferable that the second electronic information communication terminal receives the physical information.

According to this configuration, for example, the physical information of the user can be confirmed using the second electronic information communication terminal of a third party living in a place remote from the user. In this way, "monitoring" can be performed so that the remotely located third party confirms the physical information of the user.

In the above-described invention, it is preferable that when it is determined that the physical information is abnormal, the server transmits abnormality information to the second electronic information communication terminal.

According to this configuration, for example, in a case where the user (parent) is an elderly, the physical information of the user (parent) can be confirmed using the second electronic information communication terminal of the third party (child) living in a place remote from the user (parent). In a case where it is determined that the physical information is abnormal, the information is transmitted to the third party (child). Accordingly, the third party can be informed that there is a change in the physical information of the user. In this way, "monitoring" can be performed so that the remotely located third party (child) confirms the physical information of the user (parent).

In the above-described invention, it is preferable that the wearable measurement instrument has a clock function. It is preferable that at a preset timing, the detection unit automatically detects the physical information of the user.

According to this configuration, for example, if a detection timing is set to 11 o'clock every day, the physical information at the set timing (11 o'clock every day) can be automatically detected.

In the above-described invention, it is preferable that the massage machine and/or the electronic information communication terminal are communicably connected to a security organization, a health-related sales organization, or a medical institution via the public network.

Here, for example, the "security organization" includes a home security organization which provides services such as a notification service when the user is absent or an emergency assisting service.

In addition, for example, the "medical institution" includes a hospital and an institution which provides a 24-hour health consultation service.

Furthermore, for example, the "health-related sales organization" includes a health docking facility sales organization, a Chinese medicine sales organization, a supplement sales organization, a home esthetics sales organization, and a food sales organization.

According to this configuration, the user can select the proper massage course and can purchase Chinese medicines or supplements from the health-related sales organization.

A case will be described where the massage machine and/or the electronic information communication terminal are communicably connected to the security organization via the public network. For example, the user wears the wearable measurement instrument on the user's body. The user can acquire the physical information of the user while wearing the wearable measurement instrument on the user's body. Therefore, when the physical information becomes abnormal (for example, in a case where the pulse suddenly rises or the pulse cannot be monitored), the security organization is automatically notified of the abnormality via the public network. The security organization dispatches the emergency assisting service for the user, based on the notification. In this way, even in a case where the physical information of the user is suddenly changed, the security organization can flexibly cope with the sudden change.

The wearable measurement instrument is connected to the electronic information communication terminal such as a smartphone. In a case where the physical information acquired by the wearable measurement instrument is abnormal, that information is transmitted to the smartphone. The security organization is notified of the information transmitted to the smartphone via the public network.

In a case where the security organization is not connected to the electronic information communication terminal such as the smartphone, a configuration may be adopted in which the information is transmitted to the massage machine so that the massage machine notifies the security organization of the information.

A case will be described where the massage machine and/or the electronic information communication terminal are communicably connected to the medical institution via the public network. For example, the user wears the wearable measurement instrument on the user's body. The user can acquire the physical information of the user while wearing the wearable measurement instrument on the user's body. Since the massage machine and/or the electronic information communication terminal are communicably connected to the medical institution via the public network, the latest physical information of the user can always be transmitted to the medical institution. Based on the latest physical information of the user, the medical institution can carry out medical examinations, diagnoses, and health consultations.

The wearable measurement instrument is connected to the electronic information communication terminal such as the smartphone. The physical information acquired by the wearable measurement instrument is transmitted to the smartphone. The information transmitted to the smartphone is transmitted to the medical institution via the public network. In a case where the acquired physical information needs attention, the user can smoothly receive various services (doctor's medical examinations) in the medical institution, based on the information transmitted to the medical institution.

A case will be described where the massage machine and/or the electronic information communication terminal are communicably connected to the health-related sales organization via the public network. For example, the user can acquire the physical information of the user while wearing the wearable measurement instrument on the user's body. Since the massage machine and/or the electronic information communication terminal are communicably connected to the health-related sales organization via the public network, the user can purchase a health-related product, based on the current physical information of the user.

The wearable measurement instrument is connected to the electronic information communication terminal such as the smartphone. The physical information acquired by the wearable measurement instrument is transmitted to the smartphone. The information transmitted to the smartphone is transmitted to the health-related sales organization via the public network. Based on the transmitted information, the health-related sales organization can encourage the user to purchase a health-related product suitable for the physical information of the user, from among health-related products on sale.

In addition, the massage machine used for the massage machine system as described above is communicably connected to the server via the public network, and the physical information transmitted from the transmission unit is received by the receiving unit.

Furthermore, the wearable measurement instrument used for the massage machine system as described above includes the detection unit that detects the physical information and the transmission unit capable of communicating with the receiving unit.

### Advantage of the Invention

According to the massage machine system as described above, for example, the user can acquire the physical information during the user's exercise or sleep by using the wearable measurement instrument. Based on the acquired physical information, the user can select the proper massage course when the user receives the massage.

### Brief Description of the Drawings

- Fig. 1: is a conceptual diagram illustrating a configuration of a massage machine system according to an embodiment of the present invention.
- Fig. 2: is a conceptual diagram illustrating the configuration of the massage machine system according to the embodiment of the present invention.
- Fig. 3: is a perspective view of a massage machine according to the embodiment of the present invention.
- Fig. 4: is a block diagram illustrating a configuration of the massage machine.
- Fig. 5: is a block diagram illustrating a configuration of a smartphone.
- Fig. 6: illustrates an example of an advice display screen displayed on a display.
- Fig. 7: illustrates an example of a massage course information screen displayed on the display.
- Fig. 8: is a conceptual diagram illustrating a configuration of the massage machine system according to the embodiment of the present invention.
- Fig. 9: is a conceptual diagram illustrating a configuration of a massage machine system according to another embodiment of the present invention.
- Fig. 10: is a conceptual diagram illustrating a configuration of a massage machine system according to still another embodiment of the present invention.
- Fig. 11: is a conceptual diagram illustrating a configuration of a massage machine system according to still another embodiment of the present invention.

### Best Mode for Carrying Out the Invention

Hereinafter, embodiments according to the present invention will be described with reference to the drawings.

### <Massage Machine System>

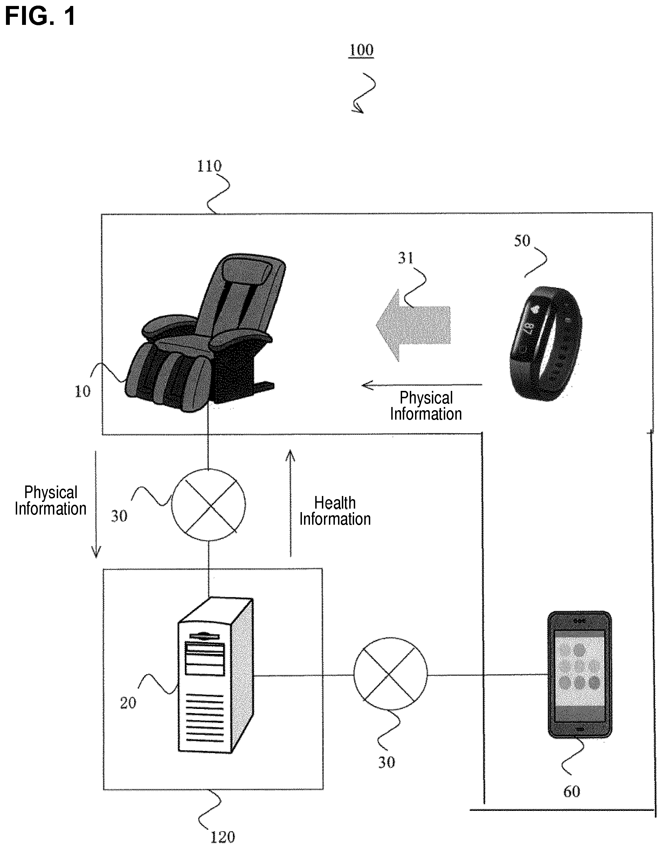

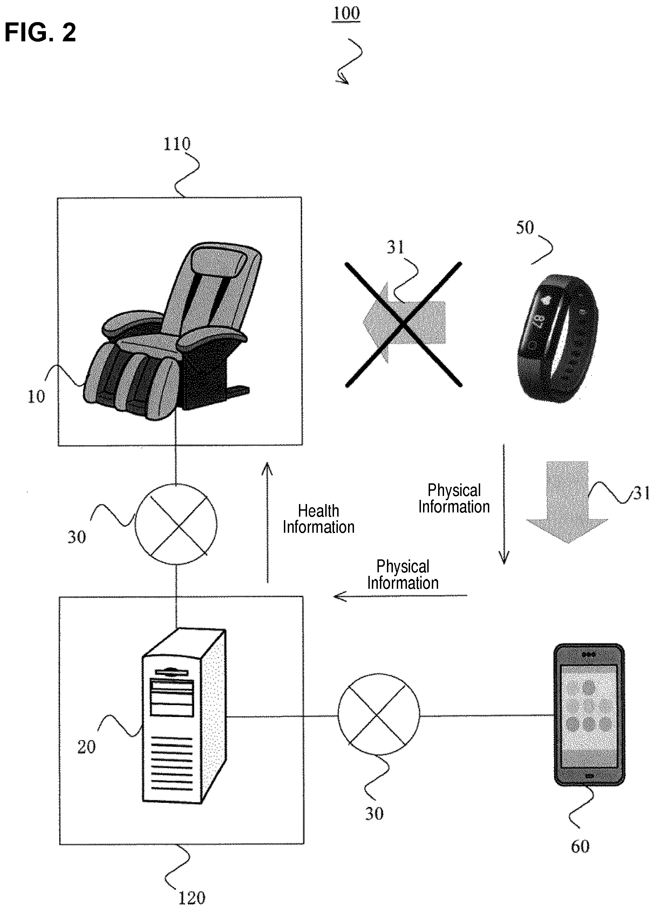

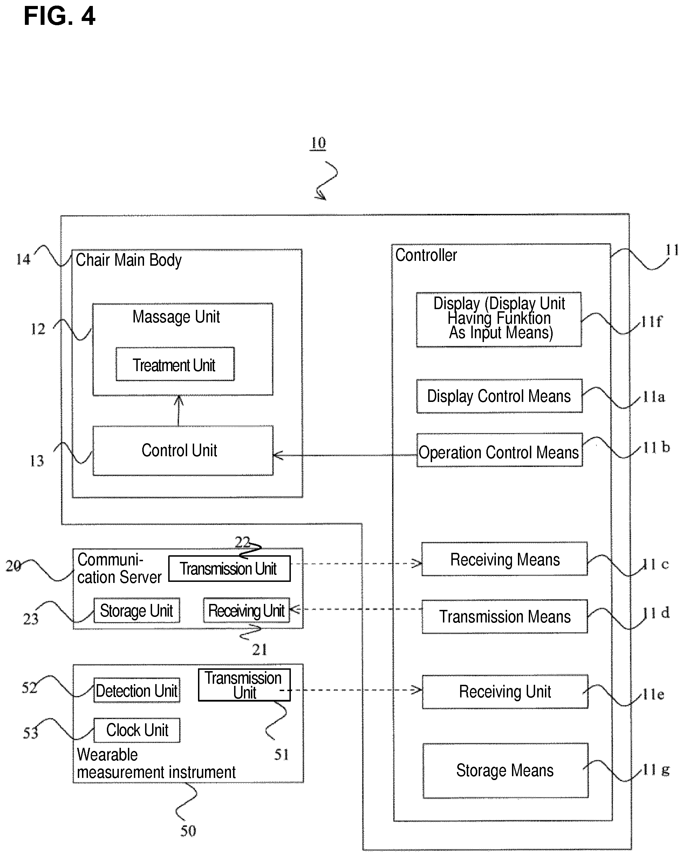

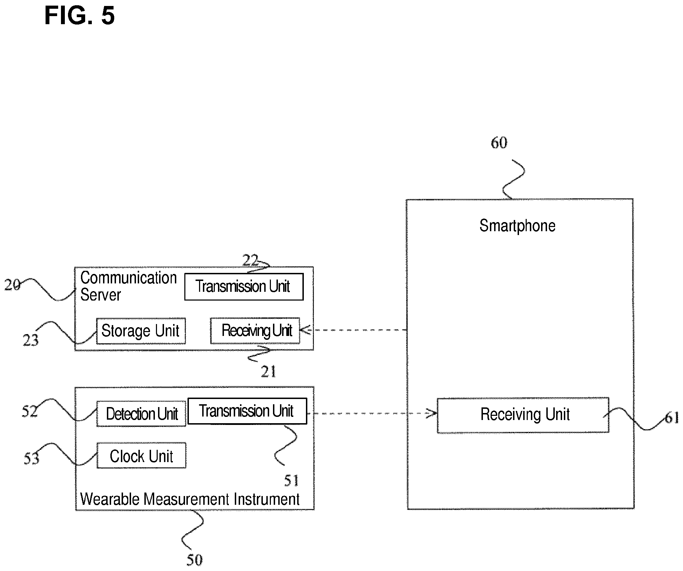

Figs. 1 and 2 are conceptual diagrams illustrating a configuration of a massage machine system according to an embodiment of the present invention. Fig. 1 is a diagram when a user is at home (distance between a wearable measurement instrument and a massage machine is 10 meters or shorter), and Fig. 2 is a diagram when the user goes out (distance between the wearable measurement instrument and the massage machine is 10 meters or longer). In addition, Fig. 4 is a block diagram illustrating a configuration of a massage machine (10), and Fig. 5 is a block diagram illustrating a configuration of a smartphone (60) serving as an electronic information communication terminal.

A massage machine system (100) includes a massage machine (10) installed at a home (110) of a user P, a cloud-type communication server (20) installed in a business operator (120), a wearable measurement instrument (50) carried by the user P, and the smartphone (60) serving as the electronic information communication terminal.

In the present embodiment, a case will be described where the smartphone (60) having a receiving unit (61) for wireless communication is used as the electronic information communication terminal. However, the present invention is not limited thereto, and a tablet terminal device may be used.

For example, the business operator includes a business operator who lends the massage machine (10), a business operator who manages the sold massage machine (10), and a hospital (medical institution). It is desirable that the hospital and the business operator cooperate with each other in order to prepare health information (to be described later).

A controller (11) of the massage machine (10) and the communication server (20) can be connected to each other using a public network (hereinafter, referred to as a "communication network") (30). The communication server (20) and the smartphone (60) can be connected to each other using the communication network (30).

For example, the communication network includes an internet network and a local network.

A transmission unit (51) of the wearable measurement instrument (50) and a receiving unit (11e) of the massage machine (10) can be connected to each other by wireless communication (31), and the transmission unit (51) of the wearable measurement instrument (50) and the receiving unit (61) of the smartphone (60) can be connected to each other by wireless communication (31).

As the above-described wireless communication, it is preferable that communication is available within a communicable distance (for example, within 10 meters). For example, the wireless communication includes the Bluetooth (registered trademark), a wireless local area network (LAN), and infrared data association (IrDA).

### <Wearable Measurement Instrument>

The wearable measurement instrument (50) can be worn on the body of the user P. For example, the wearable measurement instrument (50) includes a wearable watch equipped with a measurement instrument, which can be worn on a wrist of the user P, or a headgear equipped with a measurement instrument, which can be worn on a head of the user P.

The wearable measurement instrument (50) includes a detection unit (52) which detects physical information, the receiving unit (11e) or the transmission unit (51) capable of communicating with the receiving unit (61) of the smartphone (60) by the wireless communication (31), and a clock unit (53).

For example, the physical information detected by the detection unit (52) includes a pulse rate, a step count, a walking distance, a blood pressure, a body temperature, a sweat rate, a blood glucose level, calorie consumption, a sleep state, a body weight, a body fat percentage, moisture content, muscle mass, bone mass, a basal metabolic rate, BMI, and a obesity index.

The clock unit (53) is configured to be able to clock the time, and the user P can set a measurement timing (for example, a measurement time).

The transmission unit (51) communicates with the receiving unit (11e) of the massage machine (10) or the receiving unit (61) of the smartphone (60). As illustrated in Fig. 1, if the massage machine (10) is located within the communicable distance, the physical information is output to the receiving unit (11e) of the massage machine (10). However, if the massage machine (10) is located outside the communicable distance, the physical information cannot be output to the receiving unit (11e) of the massage machine (10). Therefore, if the massage machine (10) does not exist within the communicable distance as illustrated in Fig. 2, the physical information is output to the receiving unit (61) of the smartphone (60) carried by the user P.

The transmission unit (51) outputs the physical information to the receiving unit (61) of the smartphone (60) if the smartphone (60) is located within the communicable distance. However, if the smartphone (60) is located outside the communicable distance, the physical information cannot be output to the receiving unit (61) of the smartphone (60). However, when the user P goes out, the smartphone (60) is carried by the user P in many cases. Therefore, the smartphone (60) is located within the communicable distance (for example, within 10 meters).

In the wearable measurement instrument (50) configured in this way, even if the user P exercises or sleeps, the physical information can be detected at the measurement time, merely by wearing the wearable measurement instrument on the body of the user P. If the smartphone (60) and the massage machine (10) are located within the communicable distance, the physical information is output to the massage machine (10). In addition, if the massage machine (10) is located outside the communicable distance, the physical information is output to the smartphone (60) carried by the user P. Furthermore, if only the massage machine (10) is located within the communicable distance, the physical information is output to the massage machine (10).

### <Communication Server>

The communication server (20) is a cloud-type communication server installed in the business operator (120).

The communication server (20) and the controller (11) of the massage machine (10) can be connected to each other by the communication network (30). The communication server (20) and the smartphone (60) can be connected to each other by the communication network (30). The communication server (20) includes a receiving unit (21) which receives electronic data from the smartphone (60) or the controller (11), a transmission unit (22) which transmits the electronic data to the controller (11), and a storage unit (23).

In this manner, as the electronic data (for example, an E-mail), the communication server (20) receives the physical information detected by the wearable measurement instrument (50) by way of the controller (11) or the smartphone (60). That is, when the wearable measurement instrument (50) outputs the physical information to the massage machine (10), the physical information is received by way of the controller (11). When the wearable measurement instrument (50) outputs the physical information to the smartphone (60), the physical information is received by way of the smartphone (60). As a result, the physical information of the user P at the measurement time is received.

The storage unit (23) stores the physical information, and the physical information received from the smartphone (60) or the controller (11) by the receiving unit (21) is stored therein together with the date and time. That is, according to the present embodiment, the physical information is accumulated and searched for in a time-dependent manner, that is, a so-called database is established.

In this way, based on the physical information stored in the storage unit (23), the health information is prepared by the communication server (20). For example, the health information includes an advice based on big data, a proper massage course, and a doctor's advice. For example, a doctor, an examination technician, or a person of a business operator performs determination such as comparison between the past physical information and the current physical information. Thereafter, in a case where it is considered that a kidney function of the user P is poor, as the health information, an advice is prepared by the doctor, and information relating to the massage course is prepared by the person of the business operator (for example, "The massage suitable for a person whose kidney function is not active is recommended to receive a course for mainly massaging triple burner meridian (SJ-5) → spleen meridian (SP-6) → bladder meridian (BL-23). The triple burner meridian (SJ-5) is an acupuncture point on the wrist, and it is expected that the point has an effect to wake up the body. The spleen meridian (SP-6) is an acupuncture point inside the ankle, and it is expected that the point improves a moisture flow. The bladder meridian (BL-23) is an acupuncture point on the waist. It is expected that the point has an effect to treat a kidney disease. In addition, you may stimulate bladder meridian (BL-10) which is supposed to have an effect to relieve the tiredness showing a representative symptom of the kidney. A recommendable course is fatigue recovery course".

The transmission unit (22) performs control so as to transmit the health information as the electronic data (for example, the E-mail) to the controller (11). In this manner, as will be described later, when receiving the massage from the massage machine (10) at a later date, the user P can select a proper massage course by confirming the health information.

In the present embodiment, an example has been described in which the E-mail is exchanged as "information" exchange between the communication server (20) and the controller (11) or the smartphone (60). However, without being limited thereto, the information exchange may be made using an alternative method.

### <Massage Machine>

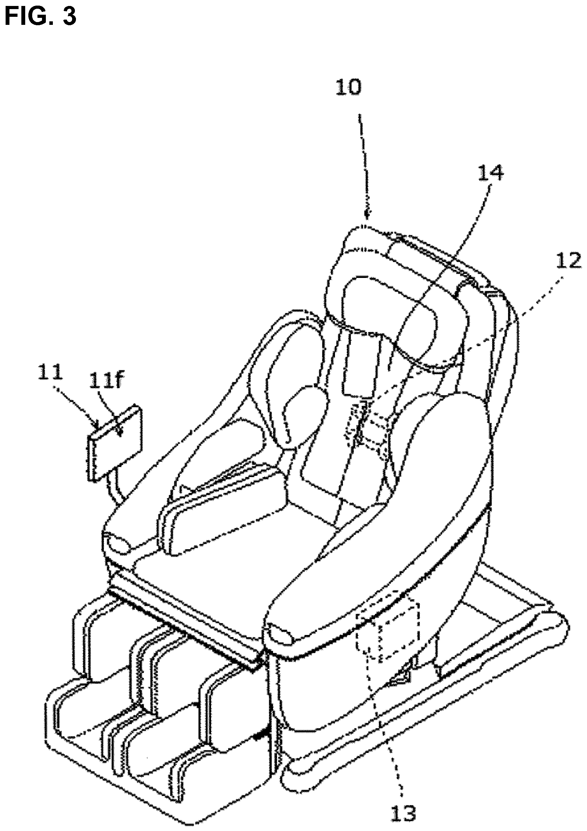

Fig. 3 is a perspective view of the massage machine according to the embodiment of the present invention. The massage machine (10) is configured to mainly include a chair main body (14) provided with a seat unit, a backrest unit, a footrest, and an armrest, a massage unit (12) provided with a motor and a treatment unit, a control unit (13) which controls the massage unit (12), and a controller (11) which receives various operation inputs relating to massage operations.

For example, the massage unit (12) is built in the backrest unit, and is provided with the treatment unit driven by the motor. The massage unit (12) is configured so that an operation position (vertical position or width position) of the treatment unit, and operation speed and a displacement amount of the treatment unit can be changed.

The control unit (13) is configured to include a CPU, a RAM, a ROM, and a memory, and receives an instruction signal from operation control means (11b) of the controller (11) when the massage is performed. Based on the instruction signal, the control unit (13) outputs a control signal so as to operate the motor and to perform the massage using the treatment unit.

The controller (11) for outputting the instruction signal to the control unit (13) is configured to include the CPU, the RAM, the ROM, and the memory. The controller (11) has a display (display unit having a function as input means) (11f), and is configured to be detachably disposed in a stand (not illustrated) on a side portion of the chair main body (14). Then, the controller (11) is connected to the control unit (13) by a signal line in a wired or wireless manner.

The storage means (11g) disposed in the controller (11) stores data relating to the massage course indicating operation patterns of the treatment unit, and stores a plurality of massage courses in advance. For example, the massage courses are used in order to perform a kneading operation, a patting operation, an acupressure operation, a rolling operation, a leg kneading operation, a pulling/kneading operation, a buttocks kneading operation, and an anus vibration operation at specified speed (without being limited to constant speed, the speed may be changed). The respective operations may be performed in combination with each other through a predetermined procedure (simultaneously or sequentially). As the massage courses, depending on the purpose of the massage, courses are prepared such as an edema relief course, a fatigue recovery course, a stress relief course, and a good night's sleep course.

For example, the "fatigue recovery course" is a massage technique which employs an idea of "massage" that is originated from ancient Europe, and which encourages blood flow from ends of the body toward the heart. Blood which is less likely to return to the heart is delivered again to the heart, fatigue recovery is encouraged, and metabolism is promoted so as to refresh the body. The "good night's sleep course" is a massage technique which employs an idea of so-called "Anma" that is originated from ancient China, and which encourages blood flow from the heart to the ends of the body. The whole blood circulation is promoted, and the blood is circulated to the ends of the body so as to provide relaxation. The massage course may include not only various operations of the massage unit (12) but also a combination of an operation for changing a reclining angle and an operation for changing a footrest angle.

Then, the controller (11) has display control means (11a) for displaying selectable massage courses or various types of information as an image on a display (11f), operation control means (11b) for receiving various operation inputs from the user P which includes operations based on the image displayed on the display (11f), and outputting an instruction signal to the control unit (13), receiving means (11c) for receiving the electronic data (for example, the E-mail) from the communication server (20), transmission means (11d) for transmitting the electronic data (for example, the E-mail) to the communication server (20), and a receiving unit (11e) to which the physical information is input from the wearable measurement instrument (50).

If the wearable measurement instrument (50) is located within the communicable distance, the receiving unit (11e) receives an input of the physical information detected by the wearable measurement instrument (50) at predetermined time intervals, and controls the storage means (11g) to store the physical information together with the date and time.

The transmission means (11d) performs control so as to transmit the physical information stored in the storage means (11g) as the electronic data (for example, the E-mail) to the communication server (20).

When the massage machine (10) is turned on, the receiving means (11c) receives the health information from the communication server (20) as the electronic data (for example, the E-mail), and controls the storage means (11g) to store the health information.

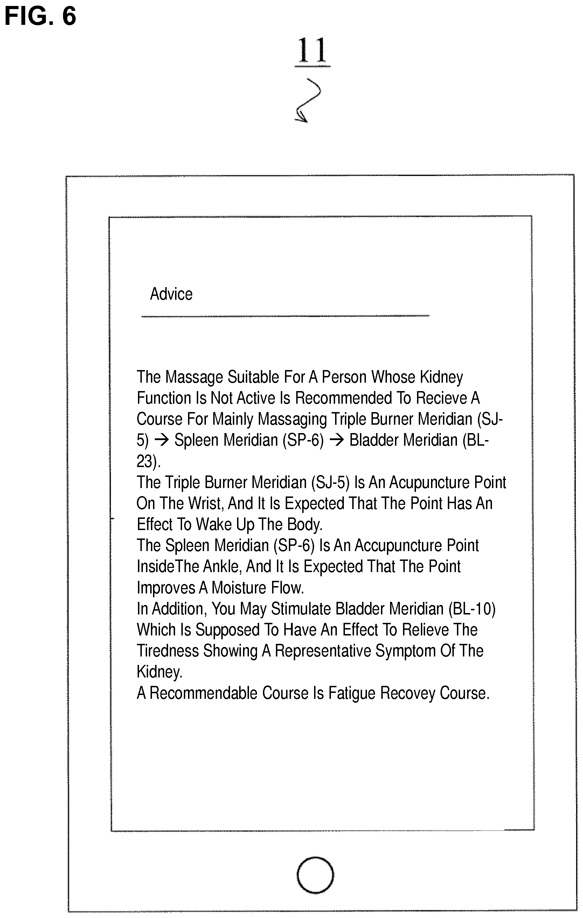

The display control means (11a) performs control so as to display a health condition of the user P on the display (11f) as an image (health condition checking function), or so as to automatically select and recommend a specific massage course, based on the health condition of the user P (massage course selecting function). For example, if the user P performs an operation in order to confirm the health condition by using the image displayed on the display (11f) before the user receives the massage, the display control means (11a) displays an advice for the health condition stored in the storage means (11g). Fig. 6 illustrates an example of an advice display screen displayed on the display (11f). The display (11f) displays a message prepared by a doctor. In this manner, in the massage machine (10) according to the present embodiment, the user P can recognize which selectable massage course is useful to the user P, and thus, can select the proper massage course. For example, the kidney function can be restored.

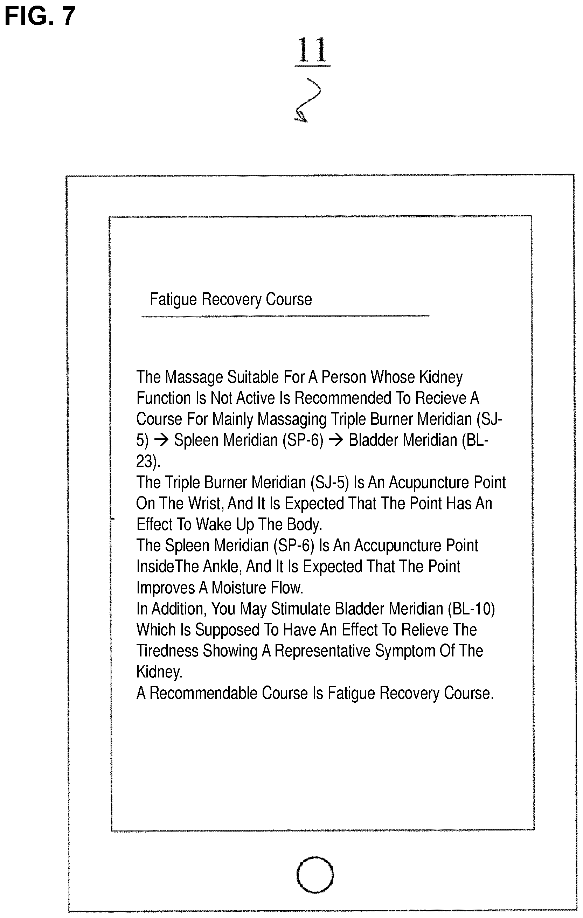

In addition, Fig. 7 illustrates an example of a massage course information screen displayed on the display. The display (11f) displays a message indicating "The massage suitable for a person whose kidney function is not active is recommended to receive a course for mainly massaging triple burner meridian (SJ-5) → spleen meridian (SP-6) → bladder meridian (BL-23). The triple burner meridian (SJ-5) is an acupuncture point on the wrist, and it is expected that the point has an effect to wake up the body. The spleen meridian (SP-6) is an acupuncture point inside the ankle, and it is expected that the point improves a moisture flow. In addition, you may stimulate bladder meridian (BL-10) which is supposed to have an effect to relieve the tiredness showing a representative symptom of the kidney. A recommendable course is fatigue recovery course". In this manner, in the massage machine (10) according to the present embodiment, the user P can recognize that the fatigue recovery course is useful to the user P. For example, the kidney function can be restored. In this case, when the recommendable course such as the fatigue recovery course is not stored in the massage machine (10) of the user P, the user P may be provided with the recommendable course by the business operator (120), or may be offered to purchase the recommendable course.

In addition, in a case where a pulse rate is high as the acquired physical information, the massage machine (10) may determine that the body of the user P is not in a good state, and the massage machine (10) may select or recommend the proper massage course (for example, a centrifugal course for lowering the pulse). In this way, the user P can receive the proper massage course, based on the acquired physical information.

Furthermore, in a case where a stress value is high as the acquired physical information, the user P himself or herself may determine that the body of the user P is not in a good state, and the user P himself or herself may be able to select the massage course (for example, the stress relief treatment). In this way, the user P can receive the proper massage course, based on the acquired physical information.

As described above, according to the massage machine system (100) in the embodiment, for example, the user P can acquire the physical information during the user's exercise or sleep by using the wearable measurement instrument (50). Based on the acquired physical information, the user P can select the proper massage course when the user P receives the massage.

### Other Embodiments

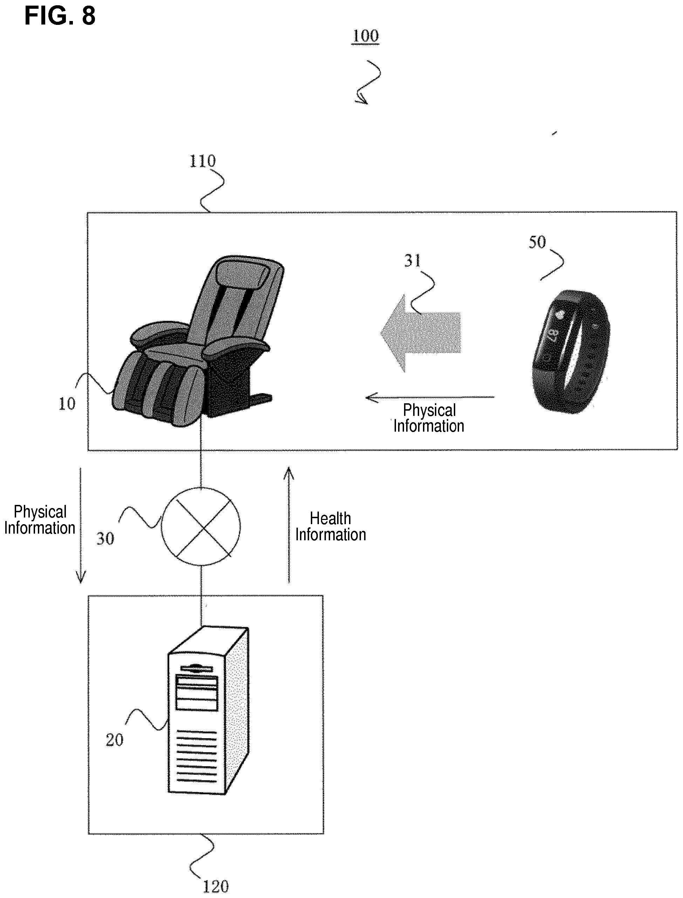

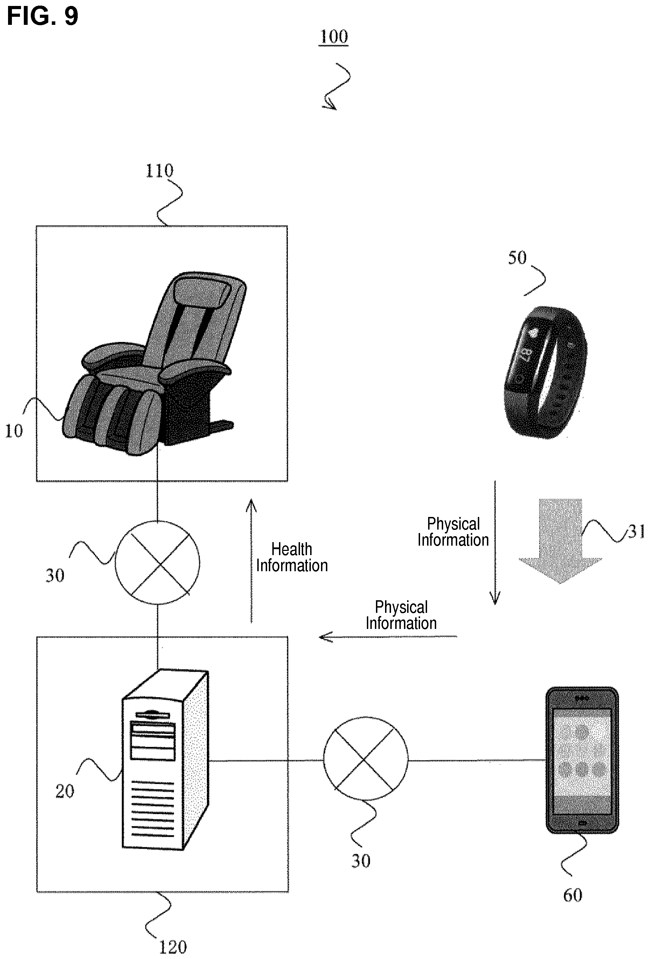

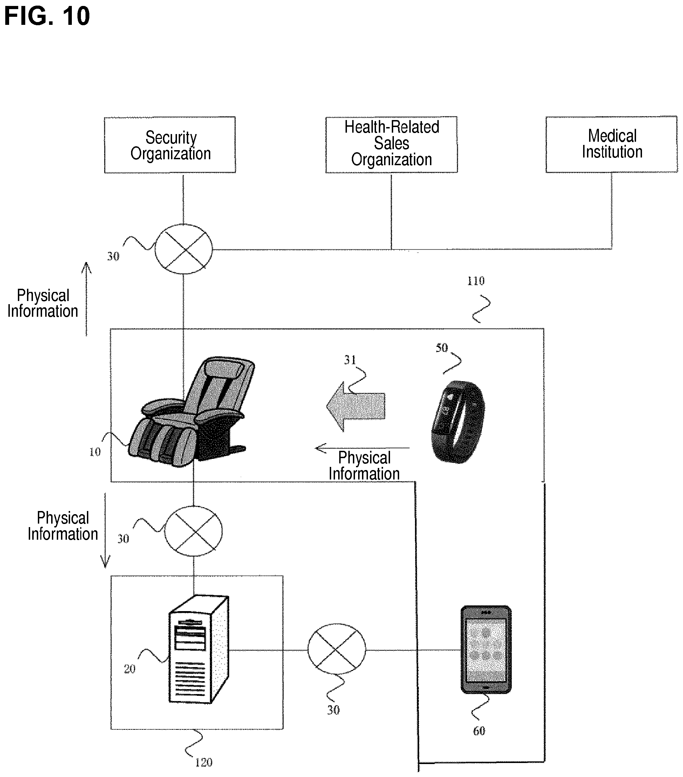

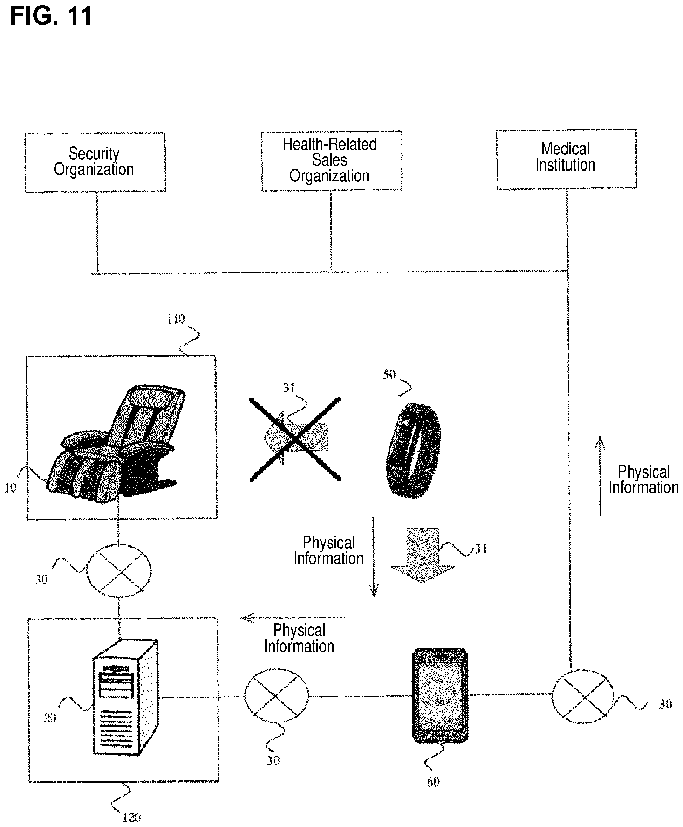

(1) In the embodiment, a case has been described where the transmission unit (22) of the communication server (20) transmits the health information to only the controller (11) by using the E-mail. However, the embodiment is not limited thereto. When it is determined that the physical information is abnormal, a configuration may be adopted so that the communication server (20) transmits the E-mail to a second smartphone carried by a third party (child) P'.
   In this way, in a case where the user P is an elderly person, the health condition of the user (parent) P can be confirmed using the second smartphone of the third party (child) P' living in a place remote from the user P. Therefore, "monitoring" can be performed so that the remotely located third party (child) P' confirms the health condition of the user (parent) P.
(2) In the embodiment, a case has been described where the massage machine system (100) includes the massage machine (10), the communication server (20), the wearable measurement instrument (50), and the smartphone (60). However, the massage machine system (100) may be configured to include the massage machine (10), the communication server (20), and the wearable measurement instrument (50). Fig. 8 is a conceptual diagram illustrating a configuration of a massage machine system according to an embodiment of the present invention. That is, the massage machine system (100) does not include the smartphone (60).
(3) In the embodiment, a case has been described where the massage machine (10) has the receiving unit (11e) to which the physical information is input from the wearable measurement instrument (50). However, the embodiment is not limited thereto. The massage machine (10) may be configured not to have the receiving unit (11e). Fig. 9 is a conceptual diagram illustrating a configuration of a massage machine system according to another embodiment of the present invention. That is, the physical information may always be received by way of the smartphone (60) and the communication server (20).
   According to the massage machine system (100) configured in this way, the user P can also acquire the physical information during the user's exercise or sleep for 24 hours by using the wearable measurement instrument (50). Based on the acquired physical information, the user can select the proper massage course when the user receives the massage.
(4) In an embodiment, a configuration may be adopted so that the controller (11) of the massage machine (10) and/or the smartphone (60) and the security organization, the health-related sales organization, or the medical institution can be connected to each other by the communication network. Figs. 10 and 11 are conceptual diagrams illustrating a configuration of a massage machine system according to another embodiment of the present invention. Fig. 10 is a diagram when the user is at home (distance between the wearable measurement instrument and the massage machine is within 10 meters). Fig. 11 is a diagram when the user goes out (distance between the wearable measurement instrument and the massage machine is 10 meters or longer).

### Industrial Applicability

The massage machine system according to the present invention is useful for the massage machine which can perform the proper massage using the physical information such as the pulse rates or the brain waves acquired by the wearable measurement instrument.

### Description of Reference Numerals and Signs

10 massage machine
11 controller
11a display control means
11b operation control means
11c receiving means
11d transmission means
11e receiving unit
11f display
11g storage means
12 massage unit
13 control unit
14 chair main body
20 communication server
50 wearable measurement instrument
51 transmission unit
60 smartphone
61 receiving unit
100 massage machine system

## Claims

1. A massage machine system comprising:
a massage machine that includes a massage unit for massaging a body of a user and a control unit for controlling an operation of the massage unit;
a wearable measurement instrument that has a detection unit for detecting physical information of the user and a transmission unit capable of transmitting the physical information detected by the detection unit;
a server for storing the physical information detected by the wearable measurement instrument; and
a portable electronic information communication terminal,
wherein the server is communicably connected to the massage machine and the electronic information communication terminal, respectively via a public network, and
wherein the electronic information communication terminal includes a receiving unit capable of receiving the physical information transmitted from the transmission unit.

2. The massage machine system according to Claim 1,
wherein the massage machine includes a receiving unit capable of receiving the physical information transmitted from the transmission unit,
wherein when the transmission unit of the wearable measurement instrument is located within a communicable distance with the receiving unit of the massage machine, the physical information is output to the receiving unit of the massage machine, and
wherein when the transmission unit of the wearable measurement instrument is located outside a communicable distance with the receiving unit of the massage machine, the physical information is output to the receiving unit of the electronic information communication terminal.

3. The massage machine system according to Claim 2,
wherein the communicable distance is 10 meters.

4. A massage machine system comprising:
a massage machine that includes a massage unit for massaging a body of a user and a control unit for controlling an operation of the massage unit;
a wearable measurement instrument that has a detection unit for detecting physical information of the user and a transmission unit capable of transmitting the physical information detected by the detection unit; and
a server for storing the physical information detected by the wearable measurement instrument,
wherein the server is communicably connected to the massage machine via a public network, and
wherein the massage machine includes a receiving unit capable of receiving the physical information transmitted from the transmission unit.

5. The massage machine system according to any one of Claims 1 to 4,
wherein the massage machine and/or the electronic information communication terminal transmit the received physical information to the server, and
wherein the server prepares health information, based on the physical information, and transmits the health information to the massage machine.

6. The massage machine system according to Claim 5,
wherein the server prepares the health information of the user, based on the stored physical information.

7. The massage machine system according to Claim 5 or 6,
wherein the health information includes an advice for diseases and/or lifestyle habits.

8. The massage machine system according to any one of Claims 1 to 7,
wherein the physical information represents at least one type of information selected from the group consisting of a pulse rate, a step count, a walking distance, a blood pressure, a body temperature, a sweat rate, a blood glucose level, calorie consumption, a sleep state, a body weight, a body fat percentage, moisture content, muscle mass, bone mass, a basal metabolic rate, BMI, and an obesity index.

9. The massage machine system according to any one of Claims 1 to 8, further comprising:
a portable second electronic information communication terminal that is provided for a third party,
wherein the second electronic information communication terminal and the server are communicably connected to each other via the public network, and
wherein the second electronic information communication terminal receives the physical information.

10. The massage machine system according to Claim 9,
wherein when it is determined that the physical information is abnormal, the server transmits abnormality information to the second electronic information communication terminal.

11. The massage machine system according to any one of Claims 1 to 10,
wherein the wearable measurement instrument has a clock function, and
wherein at a preset timing, the detection unit automatically detects the physical information of the user.

12. The massage machine system according to any one of Claims 1 to 11,
wherein the massage machine and/or the electronic information communication terminal are communicably connected to a security organization, a health-related sales organization, or a medical institution via the public network.

13. A massage machine used for the massage machine system according to any one of Claims 1 to 12,
wherein the massage machine is connected to the server via the public network, and
wherein the physical information transmitted from the transmission unit is received by the receiving unit.

14. A wearable measurement instrument used for the massage machine system according to any one of Claims 1 to 12, comprising:
the detection unit that detects the physical information; and
the transmission unit capable of communicating with the receiving unit.
